# EUROPÄISCHE PATENTSCHRIFT

(11) **EP 1 590 492 B1**
(45) Veröffentlichungstag und Bekanntmachung des Hinweises auf die Patenterteilung: **15.04.2009**
(21) Anmeldenummer: 04704197.5
(22) Anmeldetag: 22.01.2004
(51) Int. Cl.: C14C 3/16, C14C 3/22, C07D 309/30

(54) **GERBSTOFFE UND KONSERVIERUNGSMITTEL**
TANNING AGENTS AND PRESERVATIVES
TANINS ET CONSERVATEURS

(30) Priorität: 28.01.2003 DE 10303311; 20.11.2003 DE 10354396
(43) Veröffentlichungstag der Anmeldung: 02.11.2005
(73) Patentinhaber: BASF SE, 67056 Ludwigshafen (DE)
(72) Erfinder: HÜFFER, Stephan, 67063 Ludwigshafen (DE); SCHERR, Günter, 67065 Ludwigshafen (DE); REESE, Oliver, 68219 Mannheim (DE)
(86) Internationale Anmeldenummer: PCT/EP2004/000491
(87) Internationale Veröffentlichungsnummer: WO 2004/067782

(56) Entgegenhaltungen:
- EP-A- 0 066 224
- US-A- 2 546 018
- US-A- 2 941 859
- US-A- 5 360 454
- R.A. BURT ET AL.: "Vinyl ether hydrolysis. XIV. 2-Methoxy-2,3-dihydropyran: concurrent reaction of vinyl ether and acetal functional groups" CAN. J. CHEM., Bd. 58, 1980, Seiten 2199-2202, XP002284867

## Beschreibung

Die vorliegende Erfindung betrifft ein Verfahren zur Herstellung von wässrigen Formulierungen, dadurch gekennzeichnet, dass man mindestens eine cyclische Verbindung der allgemeinen Formel I 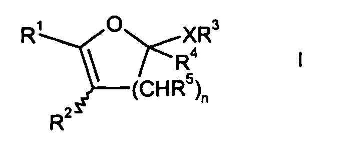 in Gegenwart von Wasser und von einem sauren Katalysator erhitzt und während des oder im Anschluss an das Erhitzen H-X-R³ zumindest partiell abtrennt, wobei in Formel I die Variablen wie folgt definiert sind:
- X: ausgewählt aus Sauerstoff, Schwefel und N-R⁶,
- R³, R⁶: gleich oder verschieden und ausgewählt aus C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, Formyl, CO-C₁-C₁₂-Alkyl, CO-C₃-C₁₂-Cycloalkyl, substituiert oder unsubstituiert, CO-C₇-C₁₃-Aralkyl, CO-C₆-C₁₄-Aryl, wobei für den Fall, dass X für N-R⁶ steht, R³ und R⁶ unter Bildung eines Rings miteinander verbunden sein können;
- R¹, R², R⁴: gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei jeweils zwei benachbarte Reste unter Bildung eines Rings miteinander verbunden sein können;
- n: eine ganze Zahl im Bereich von 1 bis 4;
- R⁵: gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei R⁵ mit R⁴ oder auch jeweils zwei benachbarte Reste R⁵ miteinander unter Bildung eines Rings verbunden sein können.

Die vorliegende Erfindung betrifft des Weiteren wässrige Formulierungen, hergestellt nach dem erfindungsgemäßen Verfahren, sowie die Verwendung der erfindungsgemäßen wässrigen Formulierungen zur Herstellung von Halbfabrikaten oder Leder und von pulverförmigen Formulierungen, die ihrerseits zur Herstellung von Leder und als Konservierungsmittel verwendet werden können. Schließlich betrifft die vorliegende Erfindung erfindungsgemäß hergestellte Leder und Halbfabrikate.

Seit über 100 Jahren ist die Chromgerbung in der Lederherstellung eine wichtige chemische Behandlung, siehe beispielsweise Ullmann's Encyclopedia of Industrial Chemistry, Band A15, Seite 259 bis 282 und insbesondere Seite 268 ff., 5. Auflage, (1990), Verlag Chemie Weinheim. Aus ökologischen Gründen wird jedoch nach Alternativen zur Chromgerbung gesucht.

Bekannt sind des Weiteren Verfahren, in denen das Chrom ganz oder teilweise durch organische Gerbmittel ersetzt wurde. Genannt seien der Einsatz der sogenannten Syntane, das sind sulfonierte Kondensationsprodukte von Formaldehyd und Phenol oder sulfonierte Naphthalin-Formaldehyd-Kondensationsprodukte. Genannt sei weiterhin der Einsatz sogenannter vegetabiler Gerbstoffe. Beide Klassen von Gerbmitteln sorgen jedoch für einen erhöhten CSB-Wert der Abwässer und sind aus Umweltgründen ebenfalls nicht unbedenklich. Außerdem hat sich erwiesen, dass die Lichtechtheit der Leder bei der Verwendung von sulfonierten Phenol/Formaldehyd-Kondensationsprodukten oft zu wünschen übrig lässt (Ullmann's Encyclopedia of Industrial Chemistry, Band A15, Seite 259 bis 282 und insbesondere Seite 270 ff., 5. Auflage, (1990), Verlag Chemie Weinheim).

Weiterhin ist die Gerbung unter Verwendung von Aldehyden, insbesondere Dialdehyden wie beispielsweise Glutardialdehyd, bekannt, siehe beispielsweise H. Herfeld, Bibliothek des Leders, Band III, Seite 191, Umschau Verlag Frankfurt/Main, 1984. Nachteilig ist jedoch, dass bei geringen Mengen an Glutardialdehyd, beispielsweise 0,5 bis 0,9 Gew.-% bezogen auf das Blößengewicht), die Schrumpfungstemperaturen nicht über 70°C hinausgehen und dass sich die erzeugten Halbfabrikate daher nur unzureichend entwässern lassen. Während des Falzens tritt eine Verleimung auf der Fleischseite des Leders auf und beeinflusst die Qualität des Leders nachteilig.

Glutardialdehyd wird in der Literatur vielfach auch als Glutaraldehyd bezeichnet, im Folgenden werden beide Ausdrücke äquivalent verwendet.

Bei größeren Mengen an eingesetztem Glutardialdehyd können Arbeitssicherheitsprobleme aufgrund der toxischen Eigenschaften des Glutardialdehyds entstehen. Außerdem wird beobachtet, dass man im Allgemeinen ausgegerbte Leder erhält und dass eine anschließende variable Verarbeitung, wie es viele Gerbereien wünschen, nicht mehr möglich ist.

Es ist bekannt, Glutardialdehyd in partiell oder ganz acetalisierter Form zum Gerben einzusetzen, beispielsweise als Methylacetal (Ullmann's Encyclopedia of Industrial Chemistry, Band A15, Seite 259 bis 282 und insbesondere Seite 273 ff., 5. Auflage, (1990), Verlag Chemie Weinheim). Die beschriebenen gegerbten Halbfabrikate neigen jedoch im Allgemeinen rasch zum Vergilben.

In DE-C 38 11 267 wird offenbart, dass eine Acetalisierung von Glutardialdehyd oder anderen Dialdehyden, die 2 bis 8 C-Atome haben, mit kurzkettigen Alkylglykolen, Alkylpolyglykolen, aliphatischen Alkoholen, Glycerin oder Sacchariden vorteilhafte Effekte bringt. Jedoch ist der Dampfdruck der Dialdehyde, die leicht aus den sehr hydrolyseempfindlichen Acetalen rückgebildet werden, noch immer merklich. Auch lassen sich die anwendungstechnischen Eigenschaften der so erhaltenen Leder weiter verbessern.

Aus EP-A 0 066 224 ist ein Verfahren zur Herstellung einer Vorstufe von Glutardialdehyd bekannt. Durch Erwärmen von 2-Alkoxy-3,4-dihydropyranen mit Säure und Wasser wird ein Gemisch aus 2-Hydroxy-6-alkoxytetrahydropyran, 2,6-Dialkoxytetrahydropyran und Glutardialdehyd erhalten, wobei die Menge an unerwünschten entstehenden oligomeren und polymeren Nebenprodukten nur gering ist (Seite 2, Zeile 33 ff.). In Gegenwart von Wasser setzen die beschriebenen Tetrahydropyranderivate Glutaraldehyd frei.

Aus US 2,546,018 ist ein Verfahren zur Herstellung von Glutaraldehyd und C-subtituierten Glutaraldehyden bekannt, das durch Hydrolyse von Glutaraldehyd in wässrigem, ggf. wässrig saurem Medium charakterisiert ist. Die durch das in US 2,546,018 offenbarte Verfahren erhältlichen Glutaraldehyd und C-substituierten Dialdehyde werden durch fraktionierte Destillation gereinigt.

Für den nach EP-A 0 066 224, DE-A 44 44 709 und US 2,546,018 erhältlichen Glutaraldehyd bzw. seine Derivate gelten die gleichen gerbereitechnischen Nachteile wie oben aufgeführt.

US 5,360,454 offenbart im Beispiel 1 ein Verfahren zur Herstellung eines Vorgerb- oder Gerbmittels durch Erhitzen von 2-Methoxy-3,4-dihydro-2-pyran in Gegenwart eines Polyethylenglykols und Säure, aber in Abwesenheit von Wasser. Man erhält mit 2-Methoxy-3,4-dihydro-2-pyran zweifach verkapptes Polyethylenglykol. Es wird kein Methanol abdestilliert.

Es bestand daher die Aufgabe, ein Verfahren bereitzustellen, durch das vielseitige wässrige Formulierungen bereit gestellt werden können. Weiterhin bestand die Aufgabe, wässrige Formulierungen bereit zu stellen, die als vielseitige Reagenzien zur Herstellung von Halbfabrikaten und von Leder geeignet sind und die aus dem Stand der Technik bekannten Nachteile vermeiden. Weiterhin bestand die Aufgabe, wässrige Formulierungen bereit zu stellen, die als Konservierungsmittel geeignet sind. Schließlich bestand die Aufgabe, Verwendungen für wässrige Formulierungen bereit zu stellen.

Demgemäß wurde das eingangs definierte Verfahren gefunden. Das erfindungsgemäße Verfahren geht aus von Verbindungen der allgemeinen Formel I 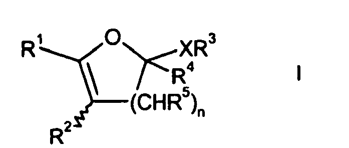 in denen die Variablen wie folgt definiert sind.
- X: ist ausgewählt aus Sauerstoff, Schwefel oder N-R⁶, wobei Sauerstoff bevorzugt ist.
- R³ und R⁶: sind gleich oder verschieden und ausgewählt aus
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl, ganz besonders bevorzugt Methyl;
C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
   unter substituierten Cycloalkylgruppen seien beispielhaft genannt: 2-Methylcyclopentyl, 3-Methylcyclopentyl, cis-2,4-Dimethylcyclopentyl, trans-2,4-Dimethylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyl;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, unsubstituiert
oder substituiertes C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, substituiert durch eine oder mehrere
   - C₁-C₁₂-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
   - Halogene wie Fluor, Chlor, Brom und Jod, wobei Chlor und Brom bevorzugt sind,
   - C₁-C₁₂-Alkoxygruppen, bevorzugt C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
Formyl,
CO-C₁-C₁₂-Alkyl wie Acetyl, Propionyl, n-Butyryl, iso-Butyryl, sec.-Butyryl, tert.-Butyryl, n-Valeroyl, iso-Valeroyl, sec.-Valeroyl, n-Capryl, n-Dodecanoyl; bevorzugt CO-C_{1-C4}-Alkyl wie Acetyl, Propionyl, n-Butyryl, iso-Butyryl, sec.-Butyryl, tert.-Butyryl, ganz besonders bevorzugt Acetyl;
CO-C₃-C₁₂-Cycloalkyl wie Cyclopropylcarbonyl, Cyclobutylcarbonyl, Cyclopentylcarbonyl, Cyclohexylcarbonyl, Cycloheptylcarbonyl, Cyclooctylcarbonyl, Cyclononylcarbonyl, Cyclodecylcarbonyl, Cycloundecylcarbonyl und Cyclododecylcarbonyl; bevorzugt sind Cyclopentylcarbonyl, Cyclohexylcarbonyl und Cycloheptylcarbonyl;
   unter substituierten Cycloalkylgruppen seien beispielhaft genannt:
   2-Methylcyclopentylcarbonyl, 3-Methylcyclopentylcarbonyl, 2-Methylcyclohexylcarbonyl, 3-Methylcyclohexylcarbonyl, 4-Methylcyclo-hexylcarbonyl, cis-2,5-Dimethylcyclohexylcarbonyl, trans-2,5-Dimethyl-cyclohexylcarbonyl, 2-Methoxycyclopentylcarbonyl, 2-Methoxycyclo-hexylcarbonyl, 3-Methoxycyclopentylcarbonyl, 3-Methoxycyclohexyl-carbonyl, 2-Chlorcyclopentylcarbonyl, 3-Chlorcyclopentylcarbonyl, 2,4-Dichlorcyclopentylcarbonyl, 2-Chlorcyclohexylcarbonyl, 3-Chlorcyclo-hexylcarbonyl, 4-Chlorcyclohexylcarbonyl, 2,5-Dichlorcyclohexylcarbonyl, 2-Thiomethylcyclopentylcarbonyl, 2-Thiomethylcyclohexylcarbonyl, 3-Thiomethyl-cyclopentylcarbonyl, 3-Thiomethylcyclohexyl;
CO-C₇-C₁₃-Aralkyl, bevorzugt CO-C₇-C₁₂-Phenylalkyl wie Phenylacetyl, w-Phenylpropionyl, besonders bevorzugt Phenylacetyl,
CO-C₆-C₁₄-Aryl, beispielsweise Benzoyl, 1-Naphthoyl, 2-Naphthoyl, 1-Anthroyl, 2-Anthroyl, 9-Anthroyl, 1-Phenanthroyl, 2-Phenanthroyl, 3-Phenanthroyl, 4-Phenanthroyl und 9-Phenanthroyl, bevorzugt Benzoyl, 1-Naphthoyl und 2-Naphthoyl, besonders bevorzugt Benzoyl.
- R¹, R², R⁴: gleich oder verschieden und ausgewählt aus Wasserstoff,
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl, ganz besonders bevorzugt Methyl;
C₃-C₁₂-Cycloalkyl, substituiert oder unsubstituiert, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
   unter substituierten Cycloalkylgruppen seien beispielhaft genannt: 2-Methylcyclopentyl, 3-Methylcyclopentyl, cis-2,4-Dimethylcyclopentyl, trans-2,4-Dimethylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyl;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl,
   unsubstituiert oder substituiertes
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, unsubstituiert oder substituiert durch eine oder mehrere
   - C₁-C₁₂-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
   - Halogene wie Fluor, Chlor, Brom und Jod, wobei Chlor und Brom bevorzugt sind,
   - C₁-C₁₂-Alkoxygruppen, bevorzugt C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
   wobei jeweils zwei benachbarte Reste unter Bildung eines Rings miteinander verbunden sein können. So können beispielsweise R¹ und R² beispielsweise gemeinsam C₁-C₈-Alkylen, unsubstituiert oder substituiert mit z.B. C₁-C₁₂-Alkyl oder C₆-C₁₄-Aryl, sein. Beispielhaft seien genannt: -CH₂-, -CH(CH₃)-, -(CH₂)₂-, -CH₂-CH(CH₃)-, -CH₂-CH(C₂H₅)-, -(CH₂)₃-, -(CH₂)₂-CH(CH₃)-, -(CH₂)₂-CH(C₂H₅)-, -(CH₂)₂-CH(C₆H₅)-, -(CH₂)₄-, -(CH₂)₅-, -(CH₂)₆-, -(CH₂)₇-, -CH(CH₃)-CH₂-CH₂-CH(CH₃)-, -CH(CH₃)-CH₂-CH₂-CH₂-CH(CH₃)-, vorzugsweise C₃-C₅-Alkylen wie beispielsweise -(CH₂)₃-, -(CH₂)₄-, -(CH₂)₅-.
- n: eine ganze Zahl im Bereich von 1 bis 4, insbesondere 2 oder 3;
- R⁵: gleich oder verschieden und ausgewählt aus Wasserstoff,
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl, ganz besonders bevorzugt Methyl;
C₃-C₁₂-Cycloalkyl, substituiert oder unsubstituiert, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl; unter substituierten Cycloalkylgruppen seien beispielhaft genannt: 2-Methylcyclopentyl, 3-Methylcyclopentyl, cis-2,4-Dimethylcyclopentyl, trans-2,4-Dimethylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyi;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-_{C14}-Aryl, substituiert oder unsubstituiert, wobei substituierte und unsubstituierte C₆-C₁₄-Arylreste wie oben stehend definiert sind.

In einer Ausführungsform der vorliegenden Erfindung können R⁵ mit R² oder R⁵ mit R⁴ oder R⁵ mit R³ oder für den Fall, dass n größer als 1 ist, jeweils zwei benachbarte Reste R⁵ miteinander unter Bildung eines Rings verbunden sein. So können R⁵ und R² beispielsweise gemeinsam C₁-C₈-Alkylen, unsubstituiert oder substituiert mit C₁-C₁₂-Alkyl oder C₆-C₁₄-Aryl, sein. Beispielhaft seien genannt: -CH₂-, -CH(CH₃)-, -(CH₂)₂-, -CH₂-CH(CH₃)-, -CH₂-CH(C₂H₅)-, -(CH₂)₃-,-(CH₂)₂-CH(CH₃)-, -(CH₂)₂-CH(C₂H₅)-, -(CH₂)₂-CH(C₆H₅)-, -(CH₂)₄-, -(CH₂)₅-, -(CH₂)₆, -(CH₂)₇-, -CH(CH₃)-CH₂-CH₂-CH(CH₃)-, - CH(CH₃)-CH₂-CH₂-CH₂-CH(CH₃)-, vorzugsweise C₃-C₅-Alkylen wie beispielsweise - (CH₂)₃-, -(CH₂)₄-, -(CH₂)₅-.

Ganz besonders bevorzugt sind die Reste R² bis R⁵ jeweils Wasserstoff. Ganz besonders bevorzugt wird als Verbindung der allgemeinen Formel 2-Methoxy-2,3-dihydro-4H-Pyran (Formel I.1) gewählt.

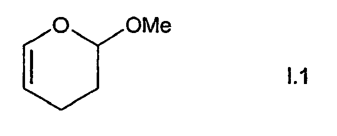

Erfindungsgemäß erhitzt man cyclische Verbindung der allgemeinen Formel I in Gegenwart von Wasser. Die Menge an Wasser kann man in weiten Bereichen wählen, bevorzugt sind 20 bis 1000 Vol.-%, bezogen auf cyclische Verbindung der allgemeinen Formel I, besonders bevorzugt sind 50 bis 500 Vol.-%, ganz besonders bevorzugt sind 60 bis 200 Vol.-%.

Das erfindungsgemäße Verfahren übt man in Gegenwart eines sauren Katalysators aus, der aus einer oder mehreren sauren Verbindungen bestehen kann.

Geeignete saure Katalysatoren sind beispielsweise Phosphorsäure, insbesondere ortho-Phosphorsäure, Ameisensäure, Essigsäure, saure Kieselgele, saures Aluminiumoxid, Schwefelsäure, Sulfonsäuren wie beispielsweise Methansulfonsäure oder para-Toluolsulfonsäure. Geeignet sind auch Mischungen aus beispielsweise Schwefelsäure und Phosphorsäure. Arbeitet man in nicht-wässrigen Lösemitteln, so ist die Anwendung von P₂O₅ oder Molekularsieb denkbar. Man kann 0,1 bis 20 Gew.-% Katalysator, bezogen auf cyclische Verbindung der allgemeinen Formel 1, einsetzen. Natürlich kann man den Katalysator oder die Katalysatoren vor dem Einsatz im erfindungsgemäßen Verfahren noch verdünnen, beispielsweise mit Wasser.

In einer Ausführungsform der vorliegenden Erfindung führt man das erfindungsgemäße Verfahren bei saurem pH-Wert durch, d.h. beispielsweise bei einem pH-Wert, der im Bereich von 0 bis 6,8, bevorzugt 0,1 bis 4 und besonders bevorzugt 0,5 bis 4 liegt.

In einer Ausführungsform der vorliegenden Erfindung übt man das erfindungsgemäße Verfahren bei Temperaturen von 40 bis 120°C, insbesondere 50 bis 85°C aus.

Man kann das erfindungsgemäße Verfahren bei beliebigen Drücken von 0,1 bis 100 bar durchführen, bevorzugt bei Atmosphärendruck.

Als Zeit für das Erhitzen sind 10 Minuten bis 24 Stunden sinnvoll, bevorzugt eine bis drei Stunden.

Erfindungsgemäß kann man in Gegenwart eines organischen Lösemittels oder eines Gemischs von organischen Lösemitteln erhitzen, beispielsweise Toluol, Petrolether oder n-Heptan, aber der Zusatz von Lösemittel ist nicht notwendig. Wünscht man Lösemittel einzusetzen, so ist eine Menge im Bereich von 10 bis 100 Vol.-%, bezogen auf cyclische Verbindung der allgemeinen Formel I, geeignet.

In einer bevorzugten Ausführungsform der vorliegenden Erfindung führt man das erfindungsgemäße Verfahren ohne Zugabe von Keton oder Monoaldehyden durch. Beispiele für Ketone sind Aceton, Methylethylketon oder Methylisobutylketon, Beispiele für Monoaldehyde sind Acetaldehyd, Benzaldehyd, Crotonaldehyd und Propionaldehyd.

Während des oder im Anschluss an das Erhitzen trennt man während des Erhitzens entstehendes H-X-R³ zumindest partiell ab.

Während der Durchführung des erfindungsgemäßen Verfahrens wird H-X-R³ gebildet, und zwar bis zu einem Äquivalent pro eingesetzter cyclischen Verbindung der allgemeinen Formel I.

In einer Ausführungsform der vorliegenden Erfindung trennt man während des Erhitzens, also noch während der Reaktion, H-X-R³ ab, beispielsweise durch Destillieren, wobei man den abgetrennte Stoffstrom, der H-X-R³ enthält und weiterhin Lösemittel enthalten kann, nur partiell oder gar nicht der Reaktionsmischung wieder zumischt.

In einer anderen Ausführungsform der vorliegenden Erfindung erhitzt man zunächst, ohne H-X-R³ abzutrennen, beispielsweise bis zum Rückfluss oder auf etwas niedrigere Temperatur als dem Rückfluss, und beginnt nach einiger Zeit mit dem Abtrennen von H-X-R³.

Wenn H-X-R³ in kristalliner Form anfällt, so kann man es durch Filtration abtrennen, vorzugsweise nach beendeter Reaktion und Abkühlen der Reaktionsmischung auf beispielsweise Raumtemperatur.

Es ist auch möglich, im Rahmen der vorliegenden Erfindung zunächst zu erhitzen, ohne H-X-R³ abzutrennen, und danach H-X-R³ zumindest partiell abzutrennen, indem man verminderten Druck anlegt.

Unter zumindest partieller Abtrennung von H-X-R³ ist im Rahmen der vorliegenden Erfindung zu verstehen, dass man mindestens 30 mol-%, bevorzugt mindestens 85 mol-% und besonders bevorzugt mindestens 95 mol-% des während des erfindungsgemäßen Verfahrens gebildeten H-X-R³ abtrennt. Man kann H-X-R³ quantitativ abtrennen, was insbesondere dann bevorzugt ist, wenn H-X-R³ toxikologisch bedenkliche Eigenschaften aufweist.

In einer Variante des erfindungsgemäßen Verfahrens trennt man bis zu 99,5 mol-% des während des Erhitzens gebildeten H-X-R³ ab, in einer weiteren Variante bis zu 99,8 mol%.

Bei der Abtrennung von H-X-R³ kann auch Wasser partiell mit abgetrennt werden. Eine quantitative Abtrennung von Wasser vermeidet man im Rahmen der vorliegenden Erfindung jedoch.

Durch das erfindungsgemäße Verfahren erhält man wässrige Formulierungen, die ebenfalls Gegenstand der vorliegenden Erfindung sind und im Folgenden auch als erfindungsgemäße wässrige Formulierungen bezeichnet werden.

Vor, während oder nach dem erfindungsgemäßen zumindest partiellen Abtrennen von H-X-R³ kann man die üblicherweise sauren erfindungsgemäßen wässrigen Formulierungen vollständig oder partiell neutralisieren. Zum Neutralisieren sind beispielsweise basische Alkalimetallsalze geeignet, wie Alkalimetallhydroxide, Alkalimetallcarbonate und Alkalimetallhydrogencarbonate. Besonders geeignet sind basische Salze von Natrium und Kalium. Geeignet sind außerdem basische Salze von Magnesium wie beispielsweise Magnesiumoxid.

In einigen Fällen wird während der Durchführung des erfindungsgemäßen Verfahrens die Bildung eines mehrphasigen Gemischs beobachtet. In den genannten Fällen ist es möglich, die jeweilige wässrige Phase durch beispielsweise Dekantieren oder andere an sich bekannte Methoden zu entfernen.

In einer bevorzugten Ausführungsform der vorliegenden Erfindung erhitzt man cyclische Verbindung der allgemeinen Formel I in Gegenwart mindestens einer Verbindung der allgemeinen Formel II 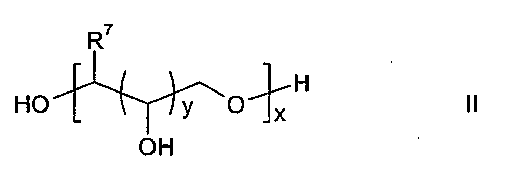 wobei in Formel II die Variablen wie folgt definiert sind:
R⁷ sind verschieden oder vorzugsweise gleich und gewählt aus
   C₁-C₄-Alkyl, Methyl, Ethyl, n-Propyl, iso-Propyl; n-Butyl, iso-Butyl, sec.-Butyl und tert.-Butyl, ganz besonders bevorzugt Methyl,
   und insbesondere Wasserstoff.
   - x: ist eine ganze Zahl im Bereich von 1 bis 250, bevorzugt 3 bis 50, besonders bevorzugt 5 bis 20.
   - y: wird gewählt aus Null oder 1.

Beispiele für Verbindungen der allgemeinen Formel II sind Ethylenglykol, Propylenglykol, Glycerin, Diethylenglykol, Dipropylenglykol, Diglycerin (R⁷ = Wasserstoff, x = 2, y = 1), Triethylenglykol, Tetraethylenglykol und Polyethylenglykole, beispielsweise Polyethylenglykole mit im Mittel 8 bis 25 Ethylenoxideinheiten (Zahlenmittel), weiterhin gemischte Polyalkylenoxide, die beispielsweise durch Reaktion von Gemischen von Ethylenoxid und Propylenoxid und gegebenenfalls Butylenoxid erhältlich sind. Besonders bevorzugt sind Polyethylenglykol mit im Mittel 7 bis 12 Ethylenoxideinheiten (Zahlenmittel) und Glycerin.

Wünscht man erfindungsgemäß in Gegenwart von mindestens einer Verbindung der allgemeinen Formel II zu erhitzen, so kann man im Bereich von 3 bis 300 Gew.-%, bevorzugt 5 bis 200 Gew.-% und besonders bevorzugt 10 bis 50 Gew.-% Verbindung der allgemeinen Formel währen, bezogen auf cyclische Verbindung der allgemeinen Formel I.

Ein weiterer Gegenstand der vorliegenden Erfindung sind wässrige Formulierungen, erhältlich nach dem erfindungsgemäßen Verfahren.

Erfindungsgemäße wässrige Formulierungen können einen Wassergehalt von 5 bis 80 Gew.-% aufweisen, bevorzugt 20 bis 50 Gew.-%. Der zu 100 Gew.-% fehlende Rest der erfindungsgemäßen Formulierungen wird im Rahmen der vorliegenden Erfindung auch als Wirkstoffgehalt der erfindungsgemäßen Formulierungen genannt, die aus der oben beschriebenen erfindungsgemäßen Umsetzung resultierenden Reaktionsprodukte, gegebenenfalls im Gemisch mit nicht umgesetzter cyclischen Verbindung der allgemeinen Formel I und gegebenenfalls im Gemisch mit Verbindung der allgemeinen Formel II, als Wirkstoffe.

Erfindungsgemäße wässrige Formulierungen können einen pH-Wert von 2 bis 9 aufweisen, bevorzugt von 3 bis 7, besonders bevorzugt 4 bis 6.

Es lässt sich beispielsweise durch Massenspektrometrie zeigen, dass erfindungsgemäße wässrige Formulierungen nur geringe Anteile an Dicarbonylverbindung der allgemeinen Formel III enthalten 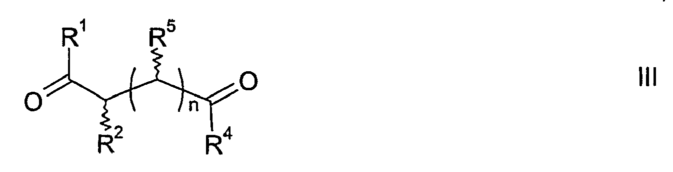 beispielsweise bis zu 10 Gew.-%, und im Wesentlichen beispielsweise durch Aldolkondensation gebildete Dimere, Oligomere und Polymere von Dicarbonylverbindung der allgemeinen Formel III. Die Variablen sind wie oben stehend definiert.

Ein weiterer Gegenstand der vorliegenden Erfindung sind Gemische von Dimeren, Oligomeren und Polymeren von Verbindung der allgemeinen Formel III, die durch geringe Anteile, beispielsweise bis zu 10 Gew.-% an Verbindung der allgemeinen Formel III verunreinigt sein können. Erfindungsgemäße Gemische von Dimeren, Oligomeren und Polymeren kann man durch das erfindungsgemäße Herstellverfahren und anschließendes Isolieren nach an sich bekannten Methoden, beispielsweise Entfernen, insbesondere Verdampfen von Wasser, erhalten. Erfindungsgemäße Gemische von Dimeren, Oligomeren und Polymeren von Verbindung III können eine breite Molekulargewichtsverteilung aufweisen, so kann das mittlere Molekulargewicht Mₙ im Bereich von 100 bis 100.000 g/mol, bevorzugt von 200 bis 10.000 g/mol liegen und der Quotient M_{w}/Mₙ im Bereich von 2 bis 20, bevorzugt von 2,1 bis 10.

Erfindungsgemäße Gemische von Dimeren, Oligomeren und Polymeren von Verbindung der allgemeinen Formel III lassen sich ohne weitere Verarbeitung oder als wässrige Formulierung zur Herstellung von Halbfabrikaten und von Leder und als Konservierungsmittel einsetzen, und zwar unmittelbar oder als pulverförmige Formulierung.

Ein weiterer Gegenstand der vorliegenden Erfindung ist die Verwendung der erfindungsgemäßen wässrigen Formulierungen zur Herstellung von Halbfabrikaten und von Leder. Ein weiterer Gegenstand der vorliegenden Erfindung ist ein Verfahren zur Herstellung von Halbfabrikaten oder Leder unter Verwendung von mindestens einer erfindungsgemäßen wässrigen Formulierung.

In einer Ausführungsform der vorliegenden Erfindung werden erfindungsgemäße wässrige Formulierungen bei der Vorgerbung, Gerbung oder Nachgerbung von Tierhäuten verwendet.

Weitere Gegenstände der vorliegenden Erfindung sind die Verwendung erfindungsgemäßer wässriger Formulierungen zum Vorgerben, Gerben oder Nachgerben von Tierhäuten sowie ein Verfahren zum Vorgerben, Gerben oder Nachgerben von Tierhäuten unter Verwendung von erfindungsgemäßen wässrigen Formulierungen.

Das erfindungsgemäße Verfahren zum Vorgerben, Gerben oder Nachgerben von Tierhäuten, im Folgenden auch erfindungsgemäßes Gerbverfahren genannt, geht aus von nach konventionellen Methoden vorbehandelten Häuten von Tieren wie beispielsweise Rindern, Schweinen, Ziegen oder Hirschen. Dabei ist es für das erfindungsgemäße Gerbverfahren nicht wesentlich, ob die Tiere beispielsweise durch Schlachten getötet wurden oder aber an natürlichen Ursachen verendet sind. Zu den konventionellen Methoden der Vorbehandlung gehören beispielsweise das Äschern, Entkälken, Beizen und Pickeln sowie mechanische Arbeitschritte, beispielsweise zur Entfleischung der Häute.

Das erfindungsgemäße Gerbverfahren wird üblicherweise in Gegenwart von Wasser durchgeführt.

Das erfindungsgemäße Gerbverfahren übt man beispielsweise so aus, dass man eine oder mehrere erfindungsgemäße wässrige Formulierungen in einer Portion oder in mehreren Portionen unmittelbar vor oder aber während eines Gerbungsschrittes zusetzt.

Das erfindungsgemäße Gerbverfahren wird vorzugsweise bei einem pH-Wert von 2,5 bis 4 durchgeführt, wobei man häufig beobachtet, dass der pH-Wert während der Durchführung des erfindungsgemäßen Gerbverfahrens um etwa 0,3 bis drei Einheiten ansteigt. Man kann den pH-Wert durch Zugabe abstumpfender Mittel um etwa 0,3 bis drei Einheiten erhöhen.

Das erfindungsgemäße Gerbverfahren übt man im Allgemeinen bei Temperaturen im Bereich von 10 bis 45°C, bevorzugt im Bereich von 20 bis 30°C aus. Bewährt hat sich eine Dauer von 10 Minuten bis 12 Stunden, bevorzugt sind eine bis 3 Stunden. Das erfindungsgemäße Gerbverfahren kann man in beliebigen gerbereiüblichen Gefäßen durchführen, beispielsweise durch Walken in Fässern oder in drehbaren Trommeln mit Einbauten.

In einer Variante des erfindungsgemäßen Gerbverfahrens setzt man eine oder mehrere erfindungsgemäße wässrige Formulierungen zusammen mit einem oder mehreren herkömmlichen Gerbstoffen ein, beispielsweise mit Chromgerbstoffen, mineralischen Gerbstoffen, Syntanen, Polymergerbstoffen oder vegetabilen Gerbstoffen, wie sie beispielsweise beschrieben sind in Ullmann's Encyclopedia of Industrial Chemistry, Band A15, Seite 259 bis 282 und insbesondere Seite 268 ff., 5. Auflage, (1990), Verlag Chemie Weinheim. Das Gewichtsverhältnis erfindungsgemäße Formulierung herkömmlicher Gerbstoff bzw. Summe der herkömmlichen Gerbstoffe beträgt zweckmäßig von 0,01 : 1 bis 100 : 1. In einer vorteilhaften Variante des erfindungsgemäßen Verfahrens setzt man nur wenige ppm der herkömmlichen Gerbstoffe den erfindungsgemäßen Formulierungen zu. Besonders vorteilhaft ist es jedoch, auf die Beimischung herkömmlicher Gerbstoffe zu erfindungsgemäßen Formulierungen ganz zu verzichten.

In einer Variante des erfindungsgemäßen Gerbverfahrens setzt man eine oder mehrere erfindungsgemäße wässrige Formulierungen in einer Portion oder in mehreren Portionen vor oder während des Vorgerbens zu, in einer besonderen Variante bereits im Pickel.

In einer weiteren Variante des erfindungsgemäßen Gerbverfahrens setzt man eine oder mehrere erfindungsgemäße wässrige Formulierungen in einer Portion oder in mehreren Portionen vor oder während eines Nachgerbungsschrittes zu. Diese Variante wird im Folgenden auch als erfindungsgemäßes Nachgerbverfahren bezeichnet. Das erfindungsgemäße Nachgerbverfahren geht aus von vorgegerbten Häuten, die auch als Halbfabrikate bezeichnet werden. Diese behandelt man mit den erfindungsgemäßen wässrigen Formulierungen.

Man kann das erfindungsgemäße Nachgerbverfahren unter ansonsten üblichen Bedingungen durchführen. Man wählt zweckmäßig einen oder mehrere, beispielsweise 2 bis 6 Einwirkschritte und kann zwischen den Einwirkschritten mit Wasser spülen. Die Temperatur bei den einzelnen Einwirkschritten beträgt jeweils von 5 bis 60°C, bevorzugt 20 bis 45°C. Man setzt zweckmäßig weitere, während der Nachgerbung üblicherweise verwendete Mittel ein, beispielsweise Fettlicker, Lederfarbstoffe oder Emulgatoren.

Ein weiterer Aspekt der vorliegenden Erfindung sind Halbfabrikate und Leder, hergestellt durch das erfindungsgemäße Gerbverfahren. Erfindungsgemäße Leder und erfindungsgemäße Halbfabrikate werden unter Verwendung von mindestens einer erfindungsgemäßen wässrigen Formulierung hergestellt. Erfindungsgemäße Leder und Halbfabrikate zeichnen sich durch eine insgesamt vorteilhafte Qualität aus, beispielsweise glatten Narben, homogenere Gerbung über den Querschnitt, verbesserte Reißfestigkeit und Fülle sowie geringere Neigung zum Verfärben, insbesondere zum Vergilben.

In einer speziellen Ausführungsform des erfindungsgemäßen Gerbverfahrens setzt man erfindungsgemäße Formulierungen in Form von pulverförmigen Formulierungen ein. Weitere Gegenstände der vorliegenden Erfindung sind daher die Verwendung von erfindungsgemäßen wässrigen Formulierungen zur Herstellung von pulverförmigen Formulierungen und ein Verfahren zur Herstellung von pulverförmigen Formulierungen unter Verwendung von erfindungsgemäßen wässrigen Formulierungen.

Zur Herstellung von erfindungsgemäßen pulverförmigen Formulierungen geht man beispielsweise so vor, dass man mindestens eine erfindungsgemäße wässrige Formulierung trocknet, beispielsweise durch Verdampfen des Wassers und besonders bevorzugt durch Sprühtrocknen.

In einer besonderen Ausführungsform der vorliegenden Erfindung vermischt man mindestens eine erfindungsgemäße wässrige Formulierung mit einem oder mehreren Zuschlagstoffen und trocknet anschließend, beispielsweise durch Verdampfen des Wassers und insbesondere durch Sprühtrocknung.

Geeignete Zuschlagstoffe sind in der Regel feste partikuläre Stoffe. Bevorzugt werden sie gewählt aus Stärke, Siliziumdioxid, beispielsweise in der Form von Kieselgel, insbesondere in der Form von sphäroidalen Kieselgelen, Schichtsilikate, Aluminiumoxid sowie Mischoxiden von Silizium und Aluminium.

### In einer speziellen Ausführungsform der vorliegenden Erfindung geht man wie folgt vor:

Es hat sich bewährt, erfindungsgemäße wässrige Formulierungen zunächst bis zu einem Restwassergehalt von 50 Gew.-% oder weniger aufzukonzentrieren. Weiterhin gibt man - so es gewünscht ist - einen oder mehrere Zuschlagstoffe zu. Anschließend entfernt man die verbliebenen flüchtigen Komponenten. Bevorzugt versprüht man die anfallenden flüssigen, festen oder öligen aufkonzentrierten Formulierungen in einem Sprühtrockner, bevorzugt in einem Sprühturm. Sprühtrockner sind dem Fachmann bekannt und beispielsweise beschrieben in Vauck/Müller, Grundoperationen chemischer Verfahrenstechnik, VCH Weinheim, 1988, 7. Auflage, S. 638-740 und S. 765-766, sowie in der darin zitierten Literatur.

Ein weiterer Gegenstand der vorliegenden Erfindung betrifft pulverförmige Formulierungen, enthaltend
10 bis 100 Gew.-%, bevorzugt 40 bis 90 Gew.-% Wirkstoff,
und 0 bis 90 Gew.-%, bevorzugt 10 bis 60 Gew.-% eines oder mehrerer Zuschlagstoffe.

Die erfindungsgemäßen pulverförmigen Formulierungen können aus feinen Partikeln mit einem mittleren Partikeldurchmesser von 100 nm bis 0,1 mm bestehen. Die Partikeldurchmesser folgen dabei einer Partikeldurchmesserverteilung, die eng oder breit sein kann. Auch bimodale Partikelgrößenverteilungen sind denkbar. Die Partikel selbst können irregulär oder sphärischer Form sein, wobei sphärische Partikelformen bevorzugt sind. Die erfindungsgemäßen pulverförmigen Formulierungen lassen sich im erfindungsgemäßen Gerbverfahren unter besonders hygienischen Verhältnissen dosieren und werden statt erfindungsgemäßer wässriger Formulierungen eingesetzt.

Es wurde weiterhin gefunden, dass erfindungsgemäße wässrige Formulierungen und erfindungsgemäße pulverförmige Formulierungen biozide Wirkung haben.

Ein weiterer Aspekt der vorliegenden Erfindung betrifft die Verwendung der erfindungsgemäßen wässrigen Formulierungen und der erfindungsgemäßen pulverförmigen Formulierungen zur Konservierung sowie Konservierungsmittel, enthaltend erfindungsgemäße pulverförmige oder wässrige Formulierung. Die erfindungsgemäßen Konservierungsmittel eignen sich zur Konservierung von Erzeugnissen, beispielsweise kosmetischen Erzeugnissen, und von Oberflächen.

Die Erfindung wird durch Beispiele erläutert.

### 1. Herstellung der erfindungsgemäßen wässrigen Formulierungen 1.1 bis 1.3 Die Molekulargewichtsbestimmungen erfolgten durch Gelpermeationschromatographie.

Stationäre Phase: mit Ethylenglykoldimethacrylat versetztes Poly-(2-hydroxyethylmethacrylat)-Gel, kommerziell erhältlich als HEMA BIO von Fa. PSS, Mainz, Deutschland.
Laufmittel: Gemisch aus 30 Gew.-% Tetrahydrofuran (THF), 10 Gew.-% Acetonitril, 60 Gew.-% 0,1 molare wässrige NaNO₃-Lösung
Interner Standard: 0,001 Gew.-% Benzophenon
Fluss: 1,5 ml/min
Konzentration: 1 Gew.-% im Laufmittel mit internem Standard
Detektion: UV/vis bei 254 nm
Kalibrierung mit Polystyrolsulfonat-Eichteils der Fa. PPS.

### 1.1. Herstellung von wässriger Formulierung 1.1

In einem 2-Liter-Dreihalskolben mit Kühler, Rührer und Thermometer wurden 200 g 2-Methoxy-2,3-dihydro-4H-Pyran (Formel I.1; 2 mol), 30 g Polyethylenglykol mit einem mittleren Molekulargewicht Mₙ von 400 g/mol, 200 ml Wasser und 6,5 g einer 50 Gew.-% wässrigen Schwefelsäure gemischt und 3 Stunden auf 77°C erhitzt. Der pH-Wert betrug 1.

Anschließend wurde auf 35°C abgekühlt und danach mit 25 Gew.-% wässriger Natronlauge ein pH-Wert von 5 eingestellt. Anschließend wurde auf 56°C erwärmt und bei einem Druck von 150 mbar 3 Stunden lang gerührt und wässriges Methanol abgetrennt.

Man erhielt 400 g erfindungsgemäße wässrige Formulierung 1.1.
Mₙ: 580 g/mol, M_{w}: 1363 g/mol, M_{w}/Mₙ: 2,58

### 1.2. Herstellung von wässriger Formulierung 1.2

In einem 1-Liter-Dreihalskolben mit Kühler, Rührer und Thermometer wurden 300 g 2-Methoxy-2,3-dihydro-4H-Pyran (Formel 1.1; 2,9 mol), 200 ml Wasser und 4,2 g 50 Gew.-% Schwefelsäure vermischt und 1 Stunde auf 80°C erhitzt. Der pH-Wert betrug 1.

Anschließend wurde auf 35°C abgekühlt und danach mit 18 g 10 Gew.-% wässriger Natronlauge ein pH-Wert von 6 eingestellt. Anschließend wurde bei 35°C bis 45°C und einem Druck von 80 bis 100 mbar 3 Stunden lang gerührt und wässriges Methanol abgetrennt.

Man erhielt 370 g erfindungsgemäße wässrige Formulierung 1.2
Mₙ: 326 g/mol, M_{w}: 660 g/mol, M_{w}/Mₙ: 2,02

### 1.3. Herstellung von wässriger Formulierung 1.3

In einem 1-Liter-Dreihalskolben mit Kühler, Rührer und Thermometer wurden 300 g 2-Methoxy-2,3-dihydro-4H-Pyran (Formel I.1; 2,9 mol), 200 ml Wasser und 6,7 g 50 Gew.-% Schwefelsäure vermischt und 1 Stunde auf 80°C erhitzt. Der pH-Wert wurde während der Reaktion durch Zugabe von weiterer Schwefelsäure bei 0,5 gehalten. Anschließend wurde auf 30°C abgekühlt und danach mit 33 g 10 Gew.-% wässriger Natronlauge ein pH-Wert von 6 eingestellt. Anschließend wurde bei 30°C und einem Druck von 60 bis 70 mbar 3 Stunden lang gerührt und wässriges Methanol abgetrennt. Man erhielt 300 g erfindungsgemäße wässrige Formulierung 1.3
Mₙ: 400 g/mol, M_{w}: 455 g/mol, M_{w}/Mₙ: 2,39

### 2. Erfindungsgemäße Gerbversuche 2.1 bis 2.3 und Vergleichsversuch

Angaben in Gew.-% beziehen sich auf das Pickelgewicht, wenn nicht anders angegeben.

Streifen mit einem Gewicht von je 2500 g gepickelter Rindsblöße mit einer Spaltstärke von 2,5 mm wurden bei einem pH-Wert von 3,0-3,2 und 25°C in einem 10-I-Fass mit 750 ml Wasser und, bezogen auf die Pickelblöße, 3 Gew.-% einer erfindungsgemäßen wässrigen Formulierung gemäß Beispiel 1.1. bis 1.3 versetzt. Nach einer Walkzeit von 60 Minuten wurden 2 Gew.-% des Sulfongerbstoffs aus EP-B 0 459 168, Beispiel K1 zugesetzt und weitere 2 Stunden gewalkt. Anschließend wurde mit 0,5 Gew.-% Magnesiumoxid innerhalb von 6 Stunden der pH-Wert auf 4,9-5,1 gestellt. Die Flotte wurde abgelassen und die Haut mit 300 ml Wasser gewaschen. Nach dem Abwelken wurden die Häute auf 1,6-1,8 mm gefalzt. Man erhielt die erfindungsgemäßen Halbfabrikate 2.1 bis 2.3.

Die Falzbarkeit wurde durch Versuche an einer Falzmaschine bestimmt. Die Falzmaschine arbeitet mit rotierenden Messern. Bei schlechter Falzbarkeit schmierten die Messer über die Oberfläche, es kam zu einer Temperaturerhöhung auf der Oberfläche der Leder, so dass ein hornartiges Aufschmelzen die Haut irreversibel schädigte. Die Benotung erfolgte nach einem Notensystem von 1 (sehr gut) bis 5 (mangelhaft).

Der Vergleichsversuch V 2.4 wurde analog durchgeführt, jedoch wurde das erfindungsgemäße Addukt durch 3 Gew.-% Glutaraldehyd (50 Gew.-% wässrige Lösung) ersetzt.

Die Schrumpftemperaturen wurden gemäß der Vorschrift aus DIN 53 336 (Jahr 1977) bestimmt, wobei die Vorschrift wie folgt modifiziert wurde:
- Punkt 4.1:: Die Probestücke hatten die Abmessungen 3 cm - 1 cm; die Dicke wurde nicht bestimmt.
- Punkt 4.2:: es wurde nur eine anstatt 2 Proben pro Ledermuster geprüft.
- Punkt 6:: entfiel
- Punkt 7:: die Trocknung im Vakuumexsikkator entfiel.
- Punkt 8:: bei Rückgang des Zeigers wurde die Schrumpfungstemperatur gemessen.

Die Benotung der Falzbarkeit und der Vergilbung erfolgte wie in der Schule: 1 sehr gut, 2 gut, 3 befriedigend, 4 ausreichend.

**Tabelle 1: Ergebnis der Gerbung und analytische Bewertung der erfindungsgemäßen Halbfabrikate**

| Nummer | Halbfabrikat | Falzbarkeit | Schrumpftemperatur [°C] | Vergilbung |
|---|---|---|---|---|
| 2.1 | 2.1 | 3 | 76 | 1,5 |
| 2.2 | 2.2 | 2 | 77,5 | 2,5 |
| 2.3 | 2.3 | 2 | 79 | 2 |
| V 2.4 | Glutaraldehyd | 4 | 77 | 4 |

### 3. Herstellung erfindungsgemäßer Leder und Vergleichsversuch

Angaben in Gew.-% beziehen sich auf das Falzgewicht, wenn nicht anders angegeben.

### 3.1. Herstellung des Leders 3.1 aus Halbfabrikat 2.1

1800 g Halbfabrikat 2.1 wurde zusammen mit den folgenden Agenzien 20 Minuten gewalkt:
120 Gew.-% Wasser, 5 Gew.-% des Sulfongerbstoffs aus EP-B 0 459 168, Beispiel K1 und
4 % einer 30 Gew.-% wässrigen, mit NaOH teilneutralisierten Lösung eines Methacrylsäure-Homopolymers mit den folgenden analytischen Daten: Mₙ ca. 10.000; K-Wert nach Fikentscher: 12 (bestimmt als 1 Gew.-% wässrige Lösung), Viskosität der 30 Gew.-% Lösung: 65 mPa·s (DIN EN ISO 3219, 23°C), pH-Wert 5,1.
Dann wurden 6 Gew.-% des Vegetabilgerbstoffs Tara® (BASF Aktiengesellschaft) und 2 Gew.-% des Harzgerbstoffs Relugan® S (BASF Aktiengesellschaft) sowie 2 Gew.-% einer wässrigen Lösung von Farbstoffen dosiert, deren Feststoffe wie folgt zusammen gesetzt waren:
70 Gewichtsteile Farbstoff aus EP-B 0 970 148, Beispiel 2.18,
30 Gewichtsteile Acid Brown 75 (Eisenkomplex), Colour Index 1.7.16.
dosiert und die Mischung gewalkt. Nach zwei Stunden wurde mit Ameisensäure der pH-Wert 3,6 eingestellt. Als Fettungskomponente wurden 6 Gew.-% eines Fettungsmittels (s. 4.) und 1 Gew.-% Lipamin OK® (BASF Aktiengesellschaft) zugesetzt. Nach einer Walkzeit von weiteren 60 Minuten wurde mit Ameisensäure der pH-Wert 3,2 eingestellt. Vor dem Ablassen der Flotte wurde eine Probe der Flotte gezogen. Man ließ die Flotte ab.

Das so erhaltene Leder wurde zweimal mit je 100 Gew.-% Wasser gewaschen, über Nacht feucht gelagert und nach dem Abwalken auf dem Spannrahmen bei 50 °C getrocknet. Man erhielt Leder 3.1. Nach dem Stollen wurde Leder 3.1 wie unten stehend beurteilt.

Die Bewertung erfolgte nach einem Notensystem von 1 (sehr gut) bis 5 (mangelhaft). Die Bewertung der Flottenauszehrung erfolgte visuell nach den Kriterien Restfarbstoff (Extinktion) und Trübung (Fettungsmittel), aus denen der Mittelwert gebildet wurde.

### Beispiele 3.2 bis 3.3, Vergleichsbeispiel V 3.4

Das obige Beispiel wurde wiederholt, jedoch jeweils mit den erfindungsgemäßen Halbfabrikaten 2.2 bis 2.3. Für das Vergleichsbeispiel V3.4 wurde das Halbfabrikat aus Vergleichsbeispiel V2.4 weiter verarbeitet. Die Bewertung der anwendungstechnischen Eigenschaften findet sich in Tabelle 2.

**Tabelle 2**

| Leder | Flottenauszehrung | Fülle | Narbenfestigkeit | Weich heit | Zugfestigkeit [N] | Stichausreißkraft [N] | Egalität |
|---|---|---|---|---|---|---|---|
| 3.1 | 2 | 2,5 | 3 | 2,5 | 268 | 186 | 2,5 |
| 3.2 | 2,5 | 2,5 | 2 | 1,5 | 264 | 190 | 2 |
| 3.3 | 2 | 1,5 | 1,5 | 2 | 280 | 194 | 1,5 |
| V 3.4 | 3 | 3,5 | 3,5 | 3 | 247 | 191 | 3 |

Die Zugfestigkeit wurde nach DIN 53328 bestimmt.

Die Stichausreißkraft wurde nach DIN 53331 bestimmt.

### 4. Herstellung des Fettungsmittels für die Beispiele 2.1 bis 2.3 und V2.4

In einem 2-1-Kessel wurden vermischt:
230 g eines Polyisobutens mit Mₙ = 1000 g/mol und M_{w} = 2000 g/mol,
30 g n-C₁₈H₃₇O-(CH₂CH₂O)₂₅-OH
5 g n-C₁₈H₃₇O-(CH₂CH₂O)₈₀-OH
40 g Ölsäure
230 g sulfitiertes oxidiertes Triolein

Die Mischung wurde unter Rühren auf 60°C erwärmt und 470 g Wasser sowie 10 g n-C₁₆H₃₃O-(CH₂CH₂O)₇-OH zugesetzt. Die entstandene Emulsion wurde anschließend durch einen Spalthomogenisiator geleitet. Man erhielt eine feinteilige, stabile Emulsion.

## Patentansprüche

1. Verfahren zur Herstellung von wässrigen Formulierungen, **dadurch gekennzeichnet, dass** man mindestens eine cyclische Verbindung der allgemeinen Formel I 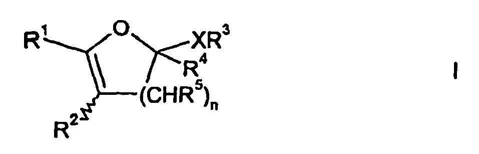 in Gegenwart von mindestens einer Verbindung der allgemeinen Formel II, 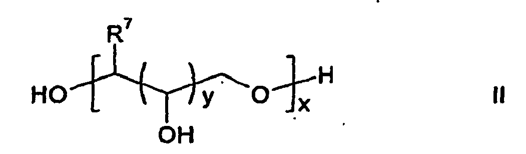 in Gegenwart von Wasser und von einem sauren Katalysator erhitzt und während des oder im Anschluss an das Erhitzen H-X-R³ zumindest partiell abtrennt, wobei in Formel 1 die Variablen wie folgt definiert sind:
X ausgewählt aus Sauerstoff, Schwefel und N-R⁶,
R³, R⁶ gleich oder verschieden und ausgewählt aus C₁-C₁₂Alkyl, C₃-C₂-Cycloalkyl, substituiert oder unsubstituiert, C₇-C₁₃-Aralkyl, C₆-C₄-Aryl, substituiert oder unsubstituiert, Formyl, CO-C₁-C₁₂-Alkyl, CO-C₃-C₁₂-Cycloalkyl, substituiert oder unsubstituiert, CO-C₇-C₁₃-Aralkyl, CO-C₆-C₁₄-Aryl, wobei für den Fall, dass X für N-R⁶ steht, R³ und R⁶ unter Bildung eines Rings miteinander verbunden sein können;
R¹, R², R⁴ gleich oder verschieden und ausgewählt aus Wasserstoff, C₁-C₂-Alkyl, C₃-C₂-Cycloalkyl, substituiert oder unsubstituiert, C₇-C₃-Aralkyl, C₆-C₁₄-Aryl, substituiert oder unsubstituiert, wobei jeweils zwei benachbarte Reste unter Bildung eines Rings miteinander verbunden sein können;
n eine ganze Zahl im Bereich von 1 bis 4;
R⁵ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei R⁵ mit R⁴ oder auch jeweils zwei benachbarte Reste R⁵ miteinander unter Bildung eines Rings verbunden sein können;
R⁷ gleich oder verschieden und gewählt aus Wasserstoff und C₁-C₄-Alkyl,
x eine ganze Zahl im Bereich von 1 bis 250;
y gewählt aus Null oder 1.

2. Verfahren nach Anspruch 1, **dadurch gekennzeichnet, dass** man es ohne Zugabe von Keton oder Monoaldehyd durchführt.

3. Verfahren nach Anspruch 1 oder 2, **dadurch gekennzeichnet, dass** X Sauerstoff bedeutet.

4. Verfahren nach einem der Ansprüche 1 bis 3, **dadurch gekennzeichnet, dass** R¹, R², R⁴ und R⁵ jeweils Wasserstoff bedeuten.

5. Verfahren nach einem der Ansprüche 1 bis 4, **dadurch gekennzeichnet, dass** n = 2 gewählt wird.

6. Wässrige Formulierungen, erhältlich nach einem Verfahren nach einem der Ansprüche 1 bis 5.

7. Verwendung von wässrigen Formulierungen nach Anspruch 6 zur Herstellung von Halbfabrikaten und von Leder.

8. Verfahren zur Herstellung von Halbfabrikaten oder Leder unter Verwendung von wässrigen Formulierungen nach Anspruch 6.

9. Verwendung von wässrigen Formulierungen nach Anspruch 6 zur Herstellung von pulverförmigen Formulierungen.

10. Verfahren zur Herstellung von pulverförmigen Formulierungen unter Verwendung von wässrigen Formulierungen nach Anspruch 6.

11. Verfahren nach Anspruch 10, **dadurch gekennzeichnet, dass** man mindestens eine wässrige Formulierung nach Anspruch 6 und gegebenenfalls einen oder mehrere Zuschlagstoffe miteinander vermischt und anschließend trocknet.

12. Verfahren nach Anspruch 11, in dem der oder die Zuschlagstoffe gewählt werden aus Stärke, Siliziumdioxid, Schichtsilikaten, Aluminiumoxid sowie Mischoxiden von Silizium und Aluminium.

13. Verfahren zur Herstellung von pulverförmigen Formulierungen nach Anspruch 11 oder 12, **dadurch gekennzeichnet, dass** man sie durch Sprühtrocknung gewinnt.

14. Pulverförmige Formulierungen, erhältlich nach einem der Ansprüche 10 bis 13.

15. Halbfabrikate und Leder, hergestellt unter Verwendung von mindestens einer wässrigen Formulierung nach Anspruch 6 oder mindestens einer pulverförmigen Formulierung nach Anspruch 14.

16. Verwendung von pulverförmigen Formulierungen nach Anspruch 14 oder von wässrigen Formulierungen nach Anspruch 6 als Konservierungsmittel.

## Claims

1. A process for the preparation of aqueous formulations, wherein at least one cyclic compound of the formula I 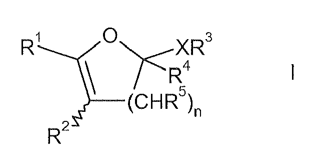 is heated in the presence of at least one compound of the formula II 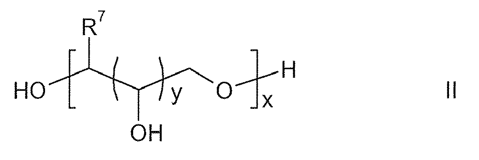 in the presence of water and of an acidic catalyst and, during or alter the heating, H-X-R³ is separated off at least partially, where, in formula I,
X is selected from oxygen, sulfur and N-R⁶,
R³ and R⁶ are identical or different and are selected from C₁-C₁₂-alkyl, C₃-C₁₂-cycloalkyl, substituted or unsubstituted, C₇-C₁₃-aralkyl, C₆-C₁₄-aryl, substituted or unsubstituted, formyl, CO-C₁-C₁₂-alkyl, CO-C₃-C₁₂-cycloalkyl, substituted or unsubstituted, CO-C₇-C₁₃-aralkyl, CO-C₆-C₁₄-aryl , where, when X is N-R⁶ , R³ and R⁶ may be linked to one another with formation of a ring;
R¹ R² and R⁴ are identical or different and are selected from hydrogen, C₁-C₁₂-alkyl, C₃-C₁₂-cycloalkyl, substituted or unsubstituted, C₇-C₁₃-aralkyl, C₆-C₁₄-aryl, substituted or unsubstituted, it being possible in each case for two neighboring radicals to be linked to one another with formation of a ring;
n is an integer from 1 to 4;
R⁵ are identical or different and are selected from hydrogen, C₁-C₁₂-alkyl, C₃-C₁₂-cycloalkyl, substituted or unsubstituted, C₇-C₁₃-aralkyl, C₆-C₁₄-aryl, substituted or unsubstituted, it being possible for R⁵ to be linked to R⁴ or in each case two neighboring radicals R⁵ to be linked to one another with formation of a ring;
R⁷ are identical or different and are selected from hydrogen and C₁-C₄-alkyl,
x is an integer from 1 to 250 and
y is selected from zero and 1.

2. The process according to claim 1, wherein it is carried out without addition of ketone or monoaldehyde.

3. The process according to claim 1 or 2, wherein X is oxygen.

4. The process according to any of claims 1 to 3, wherein R¹, R², R⁴ and R⁵ are each hydrogen.

5. The process according to any of claims 1 to 4, wherein n is chosen to be 2.

6. An aqueous formulation obtainable by a process according to any of claims 1 to 5.

7. The use of an aqueous formulation according to claim 6 for the production of semifinished products and of leather.

8. A process for the production of semifinished products or leather using an aqueous formulation according to claim 6.

9. The use of the aqueous formulation according to claim 6 for the preparation of pulverulent formulations.

10. A process for the preparation of pulverulent formulations using an aqueous formulation according to claim 6.

11. The process according to claim 10, wherein at least one aqueous formulation according to claim 6 and, if appropriate, one or more additives are mixed with one another and then dried.

12. The process according to claim 11, in which the additive or additives are selected from starch, silica, sheet silicates, alumina and mixed oxides of silicon and aluminum.

13. A process for the preparation of a pulverulent formulation according to claim 11 or 12, which is obtained by spray-drying.

14. A pulverulent formulation obtainable according to any of claims 10 to 13.

15. A semifinished product or leather produced using at least one aqueous formulation according to claim 6 or at least one pulverulent formulation according to claim 14.

16. The use of a pulverulent formulation according to claim 14 or of an aqueous formulation according to claim 6 as a preservative.

## Revendications

1. Procédé pour la préparation de formulations aqueuses, **caractérisé en ce qu'**on chauffe au moins un composé cyclique de formule générale 1 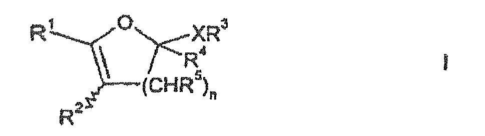 en présence d'au moins un composé de formule générale II, 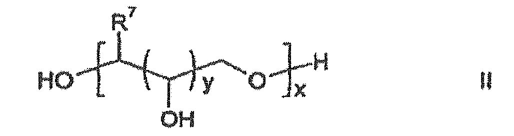 en présence d'eau et d'un catalyseur acide et on sépare pendant ou après le chauffage, au moins partiellement, H-X-R³, les variables dans la formule 1 étant définie comme suait :
X est choisi parmi l'oxygène, le soufre et N-R⁶,
R³, R⁶ sont identiques ou différents et choisis parmi C₁-C₁₂-alkyle, C₃-C₁₂-cycloalkyle, substitué ou non substitué, C₇-C₁₃-aralkyle, C₆-C₁₄-aryle, substitué ou non substitué, formyle, CO-C₁-C₁₂-alkyle, CO-C₃-C₁₂-cycloalkyle, substitué ou non substitué, CO-C₇-C₁₃-aralkyle, CO-C₆-C₁₄-aryle, où, pour le cas où X représenterait N-R⁶, R³ et R⁶ peuvent être liés l'un à l'autre en formant un cycle ;
R¹, R², R⁴ sont identiques ou différents et choisis parmi hydrogène, C₁-C₁₂-alkyle, C₃-C₁₂-cycloalkyle, substitué ou non substitue, -C₇-C₁₃-aralkyle, C₅-C₁₄-aryle, substitué ou non substitué, où à chaque fois deux radicaux adjacents peuvent être liés l'un à l'autre en formant un cycle ;
n vaut un nombre entier de la plage de 1 à 4 ;
R⁵ est identique ou différent et choisi parmi hydrogène, C₁-C₁₂-alkyle, C₃-C₁₂-cycloalkyle, substitué ou non substitué, C₇-C₁₃-aralkyle, C₆-C₁₄-aryle, substitué ou non substitué, où R⁵ peut être lié à R⁴ ou également à chaque fois deux radicaux R⁵ adjacents peuvent être liés l'un à l'autre en formant un cycle ;
R⁷ est identique ou différent et choisi parmi hydrogène et C₁-C₄-alkyle,
x vaut un nombre entier de la plage de 1 à 250 ;
y est choisi entre zéro ou 1.

2. Procédé selon la revendication 1, **caractérisé en ce qu'**on le réalise sans addition de cétone ou de monoaldéhyde.

3. Procédé selon la revendication 1 ou 2, **caractérisé en ce que** X signifie oxygène.

4. Procédé selon l'une quelconque des revendications 1 à 3, **caractérisé en ce que** R¹ R², R⁴ et R⁵ signifient à chaque fois hydrogène.

5. Procédé selon l'une quelconque des revendications 1 à 4, **caractérisé en ce qu'**on choisit n = 2.

6. Formulations aqueuses pouvant être obtenues selon un procédé selon l'une quelconque des revendications 1 à 5.

7. Utilisation de formulations aqueuses selon la revendication 6 pour la production de produits semi-finis et de cuir.

8. Procédé pour la production de produits semi-finis ou de cuir avec utilisation de formulation aqueuses selon la revendication 6.

9. Utilisation de formulations aqueuses selon la revendication 6 pour la production de formulations sous forme de poudre.

10. Procédé pour la production de formulations sous forme de poudre avec utilisation de formulations aqueuses selon la revendication 6.

11. Procédé selon la revendication 10, **caractérisé en ce qu'**on mélange au moins une formulation aqueuse selon la revendication 6 et le cas échéant un ou plusieurs adjuvants l'un avec l'autre, puis on les sèche.

12. Procédé selon la revendication 11, dans lequel le ou les adjuvants sont choisis parmi l'amidon, le dioxyde de silicium, les silicates à couches, l'oxyde d'aluminium ainsi que les oxydes mixtes de silicium et d'aluminium.

13. Procédé pour la préparation de formulations sous forme de poudre selon la revendication 11 ou 12, **caractérisé en ce qu'**on les obtient par séchage par pulvérisation.

14. Formulations sous forme de poudre, pouvant être obtenues selon l'une quelconque des revendications 10 à 13.

15. Produits semi-finis et cuir, préparés avec utilisation d'au moins une formulation aqueuse selon la revendication 6 ou d'au moins une formulation sous forme de poudre selon la revendication 14.

16. Utilisation de formulations sous forme de poudre selon la revendication 14 ou de formulations aqueuses selon la revendication 6 comme agents de conservation.
